# NEUE EUROPÄISCHE PATENTSCHRIFT

(11) **EP 1 100 479 B2**
(45) Veröffentlichungstag und Bekanntmachung des Hinweises auf die Entscheidung über den Einspruch: **07.12.2016**
(45) Hinweis auf die Patenterteilung: 11.01.2006
(21) Anmeldenummer: 99940159.9
(22) Anmeldetag: 05.08.1999
(51) Int. Cl.: A61K 9/70, A61L 29/00

(54) **MEDIZINPRODUKTE MIT RETARDIERTER PHARMAKOLOGISCHER AKTIVITÄT UND VERFAHREN ZU IHRER HERSTELLUNG**
MEDICINAL PRODUCTS WITH RETARDED PHARMACOLOGICAL ACTIVITY AND METHOD FOR THE PRODUCTION THEREOF
PRODUITS A USAGE MEDICAL POSSEDANT UNE ACTIVITE PHARMACOLOGIQUE RETARD ET PROCEDE DE FABRICATION ASSOCIE

(30) Priorität: 06.08.1998 DE 19835546; 06.08.1998 EP 98114781; 06.08.1998 US 95562 P
(43) Veröffentlichungstag der Anmeldung: 23.05.2001
(73) Patentinhaber: Schierholz, Jörg, Dr.Dr., 51427 Bergisch Gladbach (DE)
(72) Erfinder: Schierholz, Jörg, Dr.Dr., 51427 Bergisch Gladbach (DE)
(74) Vertreter: dompatent von Kreisler Selting Werner - Partnerschaft von Patent- und Rechtsanwälten mbB
(86) Internationale Anmeldenummer: PCT/EP1999/005685
(87) Internationale Veröffentlichungsnummer: WO 2000/007574

(56) Entgegenhaltungen:
- EP-A- 0 184 629
- EP-A- 0 328 421
- WO-A-96/33670
- WO-A-96/38174
- WO-A-97/25085
- US-A- 4 713 239

## Beschreibung

Gegenstand der vorliegenden Erfindung sind Medizinprodukte mit retardierter pharmakologischer Aktivität sowie Verfahren zu ihrer Herstellung.

DE-A-36 13 213 offenbart ein resorbierbares poröses Tricalciumphosphat mit einer Mischung aus einem Antibiotikum und einem weiteren Füllstoff, insbesondere einer hydrophilen Aminosäure, als besonders vorteilhaft bei der Herstellung von Knochenzementen. Hierbei wird ein niedermolekularer Zusatz in das Tricalciumphosphat hineingegeben: Glycin, welches wasserlöslich ist und welches die Wirkstoffabgabe aus dem porösen Tricalciumphosphat beschleunigt.

WO 84/03623 offenbart Magnesiumoxid, -hydroxid oder andere Magnesiumsalze, welche Effekte auf die Freisetzung von Tabletten mit Calciumantagonisten haben.

WO 89/09626 offenbart einen Hydrogel-beschichteten Schlauch, der mit Polymyxin beladen wird und verhindern soll, daß Polymyxin-sensitive Mikroorganismen Thromben formieren oder eine Infektion induzieren.

US-A-4,612,337 beschreibt eine Methode, in der Kunststoffmaterialien in Lösungen mit verschiedenen Wirkstoffen eingelegt werden, beispielsweise Ethanol, Chloroform, und später die somit oberflächlich beschichteten Materialien getrocknet werden und ein Short-term-release an Wirkstoffen induzieren.

EP-A-0 520 160 beschreibt das Quellen von Polyurethankathetern mit fluorierten Kohlenwasserstoffen, in welche durch solch einen Prozeß quartemäre Ammoniumsalze inkorporiert werden. Nach Trocknung sollen die Katheter durch Freisetzung antimikrobiell wirksam werden.

DE-A-37 25 728 beschreibt ein Verfahren, bei dem Silbermetallionen in Polyurethansilikon-Mischungen gegeben werden. Hierbei soll durch die Mischung von Polyurethanen mit Silikon Körperflüssigkeit in das Innere der Kunststoffmischung gelangen und so die Silberionen herauswaschen, welches einen besseren Keimtötungseffekt erzielen soll.

WO 89/04628 offenbart die Behandlung von Silikonimplantaten mit Antibiotika und Chloroform derart, daß die Wirkstoffe in die äußeren Schichten des Implantatmateriales hineindiffundieren können und somit für eine kurzfristige Abgabe an Wirkstoff nach Implantation des fertigen Devices gesorgt wird.

WO 87/03495 beschreibt genau das gleiche Verfahren wie WO 89/04628, mit einer Zeichnung, wie Katheter imprägniert werden können.

EP 0 484 057 A2 beschreibt einen wäßrigen Aufstrich, mit welchem Medizinprodukte antithrombogen werden sollen, indem organische Polymere, Bindungsstoffe, Spacer und Heparin als antithrombogenes Agens mit Methylenacrylsäure-Copolymer und kolloidalem Silikaten vermischt werden. Dieses Harz kann auch vorher sulfoniert werden, bevor dieser Harzanstrich auf die Medikalprodukte aufgebracht wird.

US-A-4,642,104 offenbart einen Urethralkatheter aus einem olefinischen Polymer oder einem Silikonmaterial, in welchem antimikrobielle Substanzen außen und innen gebunden werden.

US-A-4,677,143 beschreibt antimikrobielle Mischungen, mit welchen Medizinprodukte beschichtet werden. Diese Mischungen bestehen aus Acrylnitritblock-Polymeren, Polyvinylchlorid, Mischungen von Polyestern und Polyurethanen, Styrolblock-Copolymeren und natürlichen Gummiarten und Polycarbonaten, Nylon und Silikonmaterialien, welche mit oligodynamischen Substanzen, also antibakteriellen Metallen, vorzugsweise Silberoxidverbindungen gemischt werden. Diese Katheter wurden in einer klinischen Prüfung mit 1.300 Patienten untersucht. Es wurde kein Unterschied zu unbeschichteten Kathetern gefunden (Riley et al., 1995, Am. J. Med.).

WO 87/01595 offenbart, wie antimikrobielle Wirkstoffe, Antibiotika zu Plastikmaterialien vor dem chemischen Polymerisationsprozess hinzugegeben werden. Hier wird der Wirkstoff vor der Polymerisation gleichförmig in dem Kunststoff verteilt. Anwendungsbeispiel: Fixateur Externe.

WO 95/04568 beschreibt Injection Ports, welche antimikrobielle Substanzen beinhalten, beispielsweise Chlorhexidin, Silbersulfadiazin und andere.

US-A-5,525,348 offenbart Beschichtungen mit Polyvinylpyrrolidon in unterschiedlichen Lösungsmitteln, worauf verschiedene andere Oligomere mit Heparinbenzalkoniumchlorid hinzugegeben werden, die ionische Surfactant-Beschichtungen ergeben.

EP 0 640 661 A2 beschreibt Mischungen von Silikon mit silberenthaltenden Zeoliten, wobei die Silberionen aus diesen Zeoliten diffundieren.

EP 0 301 717 B1 beschreibt ebenfalls Zeolite, die in medizinische Kunststoffe eingearbeitet werden. Diese Zeolite beinhalten Silber, Kupfer oder Zink, welche eingeknetet werden und den antimikrobiellen Effekt erzielen sollen.

EP 0 470 443 B1 offenbart, wie bei niedrigen Extrusionstemperaturen dünne Coatingschichten beispielsweise auf Kathetermaterialien zusammen mit temperatursensiblen pharmazeutischen Inhaltsstoffen aufgebracht werden.

US-A-5,451,424 beschreibt Laminationsverfahren über Extrusion von Kunststoffen, mit welchen medizinische Produkte mit Arzneimitteln außen und innen beschichtet werden.

WO 92/01542 beschreibt, wie über "Solvent Casting", d.h. zur Lösung von Kunststoffen und dem gleichzeitigen Lösen von Arzneimitteln und das Eintauchen der Medikalprodukte, Beschichtungen für Nasoothopharyngialtuben hergestellt werden, beispielsweise mit dem Zweck, chronische Infektionen und Inflammationen des umgebenden Gewebes zu verhindern.

WO 96/39200 beschreibt, daß mit "Solvent Casting" ein Metallsalz in eine Kunststoffmatrix inkoorporiert werden kann.

US-A-4,999,210 beschreibt, wie eine homogene Schmelze aus Kunststoffen und Chlorhexidin hergestellt wird und über einen 2-Schrauben Compounder die Mischung auf medizinische Artikel koextrudiert wird. Später werden diese Artikel noch einmal in eine Dipping-Lösung (solvent casting) mit einem hydrophilen Polymer gegeben.

EP 0 350 161 A2 offenbart, wie mit einem Ammoniumsalz ein Substrat beschichtet und dann bei alkalischem pH-Wert heparinisiert wird.

WO 91/01767 beschreibt, wie über "Solving Casting", also (Dichlor-methan) Isopropanol und anderen Lösungsmitteln, Ammoniumheparinkomplexe an medizinische Kunststoffe gebracht werden.

EP 0 231 573 B1 beschreibt, daß pharmazeutische Substanzen entweder bei Solving Casting oder Adsorption durch Quellung in feste Kunststoffe inkoorporiert werden.

US-A-5,514,673 beschreibt eine pharmazeutische Zusammenstellung, in der über eine *transmuskosale* Applikation von Progesteron von 17-Beta-Estradiol klinische Effekte erzielt werden. Hierbei werden 3 µg bis 0,5 mg 17-Beta-Estradiol bzw. Progesteron mit natürlichen Ölen vermischt und Ei-Lecithin hinzugegeben. Diese Mischungen sollen über die Nase appliziert werden und dort Effekte ausüben. Mit dieser Methode sollen Hautirritationen vermindert werden, wobei das Lecithin als Löslichkeitsvermittler wirkt.

WO 96/32907 beschreibt einen metallischen Stent mit einer hydrophoben elastomeren Kunststoffschicht, in welche eine biologisch aktive Substanz inkoorporiert ist. Die Methode beinhaltet das "Solvent Casting". Heparin ist hier bevorzugt mit 10 bis 45% Gewicht, mit einer Schichtdicke zwischen 30 und 150 µm, bzw. Dexamethason mit einem Anteil zwischen 0,5 und 10%.

WO 96/39215 beschreibt, wie ein Silikonschlauch aufgebaut wird, welcher im Inneren des Materials eine Schicht mit pharmakologisch aktiven Reagenzien aufweist, welche langsam durch die nicht-beschichtete Außenschicht nach außen diffundieren. Über eine Silikonextrusionsmaschine wird ein Sandwich-Katheter aufgebaut. Rifampicin und Minocyclin werden hier als Puder in die Zwischenschicht eingegeben.

WO 96/33670 offenbart, wie antimikrobielle Mischungen, vorzugsweise Rifampicin, Minocyclin-Mischungen über Quellung und Erhöhung der Temperatur mit anschließender Alkalisierung der Lösung in medizinische Kunststoffe eingebracht werden.

WO 94/10838 beschreibt Lösungen mit Minocyclin-EDTA, mit denen Medikalprodukte gespült werden, um damit Infektionen zu verhindern.

US-A-5,688,516 beschreibt Mischungen aus pharmazeutischen Substanzen, welche die oben genannten drei Gruppen an Wirkstoffen enthalten sollen: Antikoagulantien, Antithrombotika und Komplexbildner wie EDTA oder DMSA. Diese Wirkstoffe sollen auf über TDMAC vorbeschichtete Medizinprodukte gebracht werden.

Die WO-A-96/33670 betrifft mit antimikrobiellen Substanzen imprägnierte Katheter und medizinische Implantate sowie ein Verfahren zur Imprägnierung dieser Medizinprodukte. Dabei wird zunächst eine antimikrobielle Zusammensetzung hergestellt mit einer wirksamen Konzentration des Wirkstoffes, um das Wachstum von Organismen zu verhindern, woraufhin diese Zusammensetzung mindestens teilweise auf das Medizinprodukt aufgebracht wird und die Zusammensetzung in das Material des Medizinproduktes permeirt. Die Zusammensetzung wird hergestellt durch Lösung des antimikrobiellen Wirkstoffs in einem organischen Lösungsmittel unter Hinzufügung eines Penetrierungsmittels und Zugabe eines alkalisierenden Stoffes.

Die WO-A-97/25085 betrifft Triclosan enthaltende Medizinprodukte.

Die US-A-4,713,239 betrifft ein Medizinprodukt bestehend aus einem Trägermaterial, das ein Homopolymer des Acrylamid oder Vinylpyrrolidons oder ein Copolymer daraus mit Arcylat ist und 99 bis 70% Acrylamid mit Vinylpyrrolidon und 1 bis 30 Gew.-% Acrylat mit einer Molmasse von 50.000 bis 1.000.000 enthält sowie ein Wirkstoff mit antianginaler Wirkung, wobei die Komponenten in nachstehenden Mengen enthalten sind: Wirksubstanz mit antianginaler Wirkung 3 bis 30 Gew.-%, biologisch löslich und resorbierbarer Träger 70 bis 97 Gew.-%.

Die EP-A-0 328 421 betrifft eine infektionsresistente Zusammensetzung, Medizinalprodukte und Oberflächen sowie Verfahren zur Herstellung und Verwendung derselben. Das dort offenbarte infektionsresistente Medizinprodukt umfaßt einen oder mehrere matrixbildende Polymere, die aus der Gruppe bestehend aus biomedizinischen Polyurethanen, biomedizinischen Silikonen und biologisch abbaubaren Polymeren sowie antimikrobiellen Wirkstoffen, insbesondere eine synergistische Kombination eines Silbersalzes und Chlorhexidin oder seines Salzes ausgewählt ist. Ebenfalls offenbart sind Medicalprodukte mit synergistischen Zusammensetzungen darin oder Zusammensetzung darauf.

Allogene Implantmaterialien sind essentiell in der modernen Medizintechnik. Die Vorteile, die mit diesen Medizinprodukten erreicht werden, also Medizinprodukten wie Peritonealkathetern, kardiovaskularen Kunststoffprodukten, orthopädischen Implantaten oder anderen prothetischen Implantaten, werden durch infektiöse Komplikationen oder thrombotische Ereignisse oder andere sekundäre Nebenwirkungen beeinträchtigt. Bei Infektionen von Kunststoffimplantaten sind die hauptverursachenden Infektionserreger Staphylococcus epidermidis und Staphylococcus aureus. Bei Gefäßkathetern sind diese Keime für 70 bis 80% aller Infektionen verantwortlich. Candida albicans ist für 10 bis 15% aller Katheterinfektionen verantwortlich. Bei Infektionen des hamableitenden Systems (UTI = Urinary Tract Infections) geht man davon aus, daß der Gebrauch von urinableitenden Kathetern wie Foley-Kathetern, suprapubischen- und Nephrostomie-Kathetern mit der Mehrzahl dieser Infektionen assoziiert ist. Solche Katheter werden häufig in ältere Patienten, Patienten mit einer Lähmung des Urogenitaltraktes, in postoperative Patienten und solche mit einer obstruktiven Uropathie implantiert.

Trotz der Berücksichtigung aller sterilen Kautelen bei Insertion und Offenhaltung all dieser Katheter bleiben diese Infektionen ein größeres Problem. In UTI sind gramnegative Bakterien mit 60 bis 70% die Hauptverursacher der Infektionen, gefolgt von Enterokokken und Candida. Im Falle metallischer Prothesen ist aufgrund der fortgeschrittenen Operationstechnik die relative Zahl an Infektionen sehr viel geringer als bei Kathetermaterialien. Da aber diese Infektionen nur durch eine Explantation der infizierten Materialien zu sanieren und die Kosten für Sanierung und Reimplantation sehr hoch sind, ganz zu schweigen, daß die Mortalität bei älteren Patienten im allgemeinen bei diesen Eingriffen ebenfalls hoch ist, sind präventive Maßnahmen ökonomisch und medizinisch betrachtet unabdingbar.

Ähnliches gilt für Gefäßprothesen; hierbei rechnet man mit Infektionsraten zwischen 1 und 3% bei inguinalen Gefäßprothesen, welche zudem nach Infektion einer bestehenden Prothese mit wesentlich höheren Infektionsraten reimplantiert werden. Hier besteht das Problem in der hohen Mortalität der Patienten mit bis zu 50% und der hohen Amputationsrate der distal betroffenen Extremität. Ein weiteres Problem besteht bei allen Medikalprodukten wie zentralen Venenkathetern, intrakoronalen Stents, Herzklappen (Olenoff (1)), resorbierbaren Wundauflagen, CAPD-Kathetern, Spinnvliesen, oder auch Gefäßprothesen in der Thrombogenität. Die thrombogene Potenz von Fremdkörpermaterialien läßt sich grundsätzlich durch die Modifikation mit antithrombogenen Arzneimitteln verringern.

Eine dritte Gruppe von Indikationsfeldern betrifft okulare Implantate, wobei hier antiinfektive und antihypertensive Mittel als Beschichtungssubstanzen vorteilhaft sind. Andere Indikationsfelder sind die Verhinderung der Inkrustation durch Komplexbildner- Freisetzung aus UT-Kathetern und die kontrollierte Abgabe von Hormonen, Antihormonen, Zytostatika und Peptiden aus Zementen, Prothesenmaterialien, Herzschrittmachern, Stents und andere Materialien.

Eine bekannte Methode zur Modifikation von Medikalprodukten beruht darauf, daß auf die Oberfläche von Kathetern eine Schicht mit Tridodecylmethylamoniumchlorid (TDMAC)-surfactant und darauffolgend antimikrobielle Substanzen aufgebracht werden, welche ionisch geladen sind. Beispielsweise können Polyethylen-Silikon-Elastomere, Polytetraflourethylen oder auch Dacronmaterialien in 5%igen TDMAC-Lösungen für 30 Minuten gebadet, getrocknet und gespült werden. Diese TDMAC-Surfactant tragende Medizinprodukte können dann in verschiedene Wirkstofflösungen inkubiert werden.

Ähnliches kann man mit Benzalkoniumchlorid und der ionischen Bindung von antimikrobiellen Substanzen durchführen (Solomon DD, Sheretz, REJ in J. Contr. Release 6:342-352 (1989) und US-Aatent Nr. 4,442,133).

Andere Methoden beinhalten ein ionisches Binden von Wirkstoffen mit negativ geladenen Gruppen an der Oberfläche von Medikalprodukten (US-A-4,895,566),
- das Anquellen von Medizinprodukten und die Adsorption in die Oberfläche (US-A-4,917,686),
   oder das Aufbauen von ionischen Hydrogelen, welche dann später ionisch gebundene Wirkstoffe tragen (US-A-4,107,121),
- bzw. das Laminieren von antimikrobiellen Substanzen an oberflächliche Schichten (US-A-5,013,306)
- oder auch nur die Anwendung eines Silikonöls auf ein Medizinprodukt und dann Inkontaktbringung des Silikonfilmes mit Wirkstofflösung (US-A-4,952,419).

Ein Nachteil dieser Methoden liegt darin, daß durch solche physiko-chemischen Methoden die Wirkstoffaktivität relativ schnell nachläßt. Hinsichtlich der antimikrobiellen Wirksamkeit ist es bespielsweise so, daß nach dem Absinken der bakteriziden Oberflächenaktivität Katheter oder Gefäßprothesen oder andere Implantate wieder bakteriell besiedelt werden können. Gleiches gilt für die Beschichtung mit antithrombogenen Mitteln. Hierbei ist klinische Effizienz nur dann zu erwarten, wenn in ausreichender Menge Wirkstoffe freigesetzt werden. Ähnliches gilt auch für antiproliferative und proliferative Substanzen. Nachteil dieser Beschichtungen ist die fehlende effiziente Kontrolle der Freisetzung der pharmazeutischen Substanzen aus den Medizinprodukten (4). Wünschenswert ist es, einer idealisierten Freisetzung nullter Ordnung sehr nahe zu kommen, um über längere Zeit hinweg konstante Wirkungen zu erzielen.

Auch Gefäßprothesen und metallische Implantate (z.B. TEP's) können mit oder ohne Spacer (Albumin, Kollagen, biodegradierbare Coatings) mit antibakteriellen, antithrombotischen, antiflammatorischen, immunmodulierenden, endokrin wirksamen Substanzen modifiziert werden.

Aufgabe der Erfindung ist die Schaffung von nicht degradierbaren Medizinprodukten, welche pharmakologisch aktiv sind, sowie Methoden bereitzustellen, mit denen möglichst lange eine pharmakologische Aktivität aufrechterhalten werden kann und so die genannten Nachteile des Standes der Technik zu überwinden. Eine weitere Aufgabe der Erfindung ist es, eine praktische, kostengünstige, sichere und effiziente Methode zur Verfügung zu stellen, um eine Vielzahl von Kathetertypen und andere Medikalprodukte mit unterschiedlichsten Substanzkombinationen zu modifizieren. Ein Ziel dieser Erfindung liegt u.a. darin, Medizinprodukte möglichst lange gegen Infektionen zu schützen, und dies mittels unterschiedlicher Inkorporierungs- und Beschichtungsmethoden.

Die Aufgabe wird gelöst durch ein nicht abbaubares Medizinprodukt gemäß Anspruch 1. Dabei kann eine geringe Löslichkeit sowohl kinetisch als auch thermodynamisch bedingt sein. Zum Beispiel wird die Löslichkeit bei Raumtemperatur (ca. 25°C), pH 7 gemessen.

Überraschenderweise wurde gefunden, daß bei einer nicht-abbaubaren polymeren Matrix eine monolithische Verteilung von zwei Substanzen mit unterschiedlicher Hydrophilie eine Retardierung der Freisetzung der hydrophilen Komponente erzielt wird, wenn eine hydrophile Substanz dispers im Polymeren (bis 10 % wt) mit einer hydrophoben Substanz (bis 10 % wt) kombiniert wird. Dieser Effekt wird physikochemisch durch die Interaktion der Wirkstoffe auf molekularer Ebene erzeugt und damit eine Erniedrigung der Diffusionsgeschwindigkeit erreicht, es gibt keinen Abbau der polymeren Matrix und damit eine weitere Beeinflussung der Freisetzung. Weiterhin überraschend war der Befund, daß die Assoziation zweier Wirkstoffe durch eine Salzbildung (Miconazol-Base-Fusidinsäure, Ag-EDTA) die Retardierung durch die lipophile Komponente verstärkt.

Nach WO-A-96/38174 können mikroverkapselte und homogen verteilte Substanzen in einer biodegradierbaren polymeren Matrix die Freisetzung kontrollieren, maßgeblich gesteuert durch die Polymermatrixdesintegration. Die Biodegradation erleichtert die Freisetzung von Substanzen durch das Aufbrechen der von molekularen Bindungen im Trägermaterial und ermöglicht die Entstehung von Poren, Hohlräumen durch das Eindringen von Außenmedium (Blut, Liquor, Urin). Die Wirkstoffe werden durch Konvektion, Degradation, Lösung und auch Diffusion aus dem Verband freigesetzt. Ist die polymere Matrix zudem kristallin, wie in WO-A-96/38174 beschrieben, ist eine Wirkstoffdiffusion durch kristallines Polylactid fast nicht möglich und somit wird die Wirkstofffreisetzung fast ausschließlich durch die Geschwindigkeit der Biodegradation gesteuert. Die Beeinflussung der Freisetzung durch die Wahl der Substanzen ist in diesem Fall erst dann möglich, wenn mehr Substanz als Matrix vorliegt (bevorzugter Bereich von 30 - 80 Massenprozent).

In der vorliegenden Erfindung ist dies aber bereits bei stabilen Medizinprodukten schon bei 1 - 20% Wirkstoffanteil möglich.

Darüberhinaus wurde überraschenderweise gefunden, daß der Retardierungseffekt durch eine Gradientenbildung der lipophilen Substanz bezogen auf das Verhältnis Oberflächenkonzentration zu Bulk-Konzentration (Matrix-Konzentration) verstärkt wurde, wenn oberflächlich mehr lipophile Substanz angelagert wurde. Dies kann beispielsweise durch eine Extraktion eines beladenen Medizinproduktes mit einer Lösung der lipophilen Komponente in einem geeigneten Solvens durchgeführt werden, wodurch oberflächlich mehr lipophile Substanz angelagert und hydrophile Substanz extrahiert wird.

Es wird offenbart, daß die schwerlösliche Substanz selbst als Trägermatrix und als Depot wirken kann zu einer Retardierung der Freisetzung (s. Beispiel 6). Somit können Lacke auf nicht-degradierbare Implantatoberflächen aufgebracht werden, welche lediglich aus einer lipophilen und hydrophilen Komponente bestehen.

Der Retardierungseffekt ist am ausgeprägtesten im Überschuß der lipophilen Komponente im Verhältnis zur hydrophilen Komponente und bei Löslichkeiten der lipophilen Komponente von 100 µg/ml bis 1 µg/ml.

Die Löslichkeit ergibt sich durch die maximale Menge einer Substanz, die bei 25°C in Wasser löslich ist. Berechnet wird das Gewicht der Substanz bezogen auf das Gesamtgewicht der erhaltenen Lösung. Im Rahmen dieser Anmeldung werden geringlösliche Substanzen auch mit der Eigenschaft "lipophil" charakterisiert.

Offenbart werden geeignete Medizinprodukte wie beispielsweise Medizinprodukte metallischer und nichtmetallischer Form wie Endoprothesen, Knieprothesen oder auch Katheter und Drainagen und andere polymere Medizinprodukte, welche mittels unterschiedlicher Methoden mit einer Kombination pharmazeutischer Substanzen bzw. Wirkstoffe modifiziert werden. Durch die Modifizierung mit pharmazeutischen Substanzen infektionsresistenter werden sollen oder b) hämo- oder biokompatibler oder c) letztendlich die Funktion des Medikalproduktes bzw. das Einwachsen in das umgebende Gewebe verbessern bzw. andere spezifische pharmakologische Effekte erzielen sollen. Wesentlich ist die Kombination zweier unterschiedlicher Substanzen für eine Beschichtung, Imprägnierung oder anderweitige Modifikation von Medizinprodukten, wobei eine Substanz eine geringe Löslichkeit in Wasser aufweist und die zweite Substanz eine höhere Löslichkeit in Wasser hat. Dies führt dazu, daß die Substanz A in dem umgebenden Medium (Liquor, Blut, Urin, Gewebe oder Peritonealflüssigkeit) eine geringere Löslichkeit aufweist als im Polymeren bzw. so schwer löslich ist, daß durch die Lösungsprozesse in unterschiedlichen Körperflüssigkeiten eine lange Freisetzungsphase ermöglicht wird, wie es auch bei nur oberflächlich beschichteten Totalendoprothesen der Fall sein kann. Die gering lösliche Substanz A sorgt überraschenderweise dafür, daß die Freisetzung der anderen Substanz B retardiert, d.h. verlangsamt wird. Durch die Verlangsamung der Freisetzung der Substanz B erhält man ein sogenanntes "steady state release"-Profil, in welchem man nach einem sehr flachen initialen Abfall der Freisetzung eine nahezu konstante Freisetzung erzielt. Die solchermaßen modifizierten Medizinprodukte sind, insbesondere wenn Substanz B ein Wirkstoff ist, in der Lage, länger pharmakologisch aktiv zu bleiben.

Die Erfindung wird im folgenden am Beispiel einer auf eine Substanz A und eine Substanz B beschränkten Kombination erläutert.

Dadurch, daß die Substanz A in dem späteren Implantationsgewebe (Blut, Liquor, Urin, Peritonealflüssigkeit, Gewebsflüssigkeit) nur sehr gering löslich ist, erhält man eine retardierte, verlängerte Freisetzung dieser Substanz. Unerwartet war der Befund, daß durch die verlangsamte Freisetzung dieser Substanz A auch die Freisetzung einer normalerweise sehr viel schneller abgegebenen Substanz B verzögert wird. Die Substanz A wirkt erstens in nicht zu erwartender Weise als Eigendepot, d.h. als Reservoir, welches sich nach und nach sehr langsam auflöst, und zweitens überraschenderweise als Diffusionsmatrix für Substanz B, die ohne die diffusionsbezogene Matrix sehr viel schneller in kurzer Zeit abgegeben werden würde. Die Löslichkeit der Substanz A darf jedoch nicht so gering sein, daß die Substanz A praktisch unlöslich ist, da dann keine pharmakologisch wirksamen Konzentrationen im umgebenden Medium erreicht werden. Ungeeignet ist beispielsweise Silberchlorid. Es kann Substanz B ein Wirkstoff sein.
Bevorzugte Wirkstoffe weisen antimikrobielle, antithrombogene, antiproliferierende, immunmodulierende und/oder endokrine Eigenschaften auf. Es hat sich bewährt, daß eine Beladung von mindestens 0,1% Masseprozent Substanzen in der Wirkstoffirägerschicht vorhanden ist, um über einen längeren Zeitraum pharmakologische Wirkungen in der Mikroumgebung des Medizinproduktes zu erzeugen. Bei der Mischung der Subtanz A mit Substanz B hat es sich bewährt, daß die Substanz A einen Anteil größer als 0, 1 % (Größenordnung) der gesamten Substanz hat. Sehr viel geringere Anteile an der Kombination haben nicht mehr die gewünschte Retardierungswirkung. Die Retardierungswirkung steigt in der Regel mit steigender Inkorporationsrate und Anteil der Substanz A.

Es wird bevorzugt, daß sich der Wirkstoff intrazellulär in den Zielorganen oder Organismen (z.B. Bakterien, Thrombozyten) anreichert.

Es ist besonders bevorzugt, daß Substanz A und B beides Wirkstoffe sind, die sich intrazellulär anreichern.

Geeignete Medizinprodukte sind Augenlinsen, Katheter, Gefäßprothesen, Endoprothesen, chirurgisch antimikrobielle Wirkstoffträger wie Kollagenvlies, Stents, Blades, Knochenzement, metallische Endoprothesen, Knieprothese, Hüftprothese, CAPD-Katheter, Wundauflagen, gesprühte Polyurethanvliese, Drainage, Knochen-Weichgewebeersatzstoffe.

Bevorzugte Materialien für die Medizinprodukte sind Siloxane, Polyurethane, Acrylate, Polycarbonate, Cellulose, Cellulose-Derivate, Polymere aus Tetrafluorethylen und Polyethylentherephthalat.

Die Substanzen können auf unterschiedlichste Art und Weise mit den Medizinprodukten kombiniert werden. Eine hocheffiziente Modifikation von Medizinprodukten findet über Quellung mittels geeigneter Lösemittel und geeigneter Quellpartner statt wie in EP 0 550 875 beschrieben. Hierbei werden aufgrund ähnlicher Löslichkeitsparameter (Hansen Parameter, Kohäsionsenergiedichten) von Polymer und pharmazeutischer Substanz ausreichend hohe Konzentrationen an Wirkstoffen in Medizinprodukten erreicht. Diese so beladenen Medizinprodukte werden mittels kontrollierter Freisetzung der Wirkstoffe aus diesen Medizinprodukten heraus pharmakologisch wirksam.

Beispiele hierfür sind die Quellung von Polyurethanen mit Alkoholen, Aceton, Ketonen, Ester wie Essigsäureethylester, bei ausgesuchten aromatischen Polyurethan mit CH₂Cl₂, DMF, DMA, Methylethylketon, HMPT, DMSO, Dioxan (heiß), n-Kresol, Tricresol, n-Propanol, Dichloressigsäure oder Mischungen davon.

Offenbarte Beispiele für Kombinationen von pharmazeutischen Wirkstoffen, Quellmitteln und Polymeren können der folgenden Tabelle entnommen werden.

| Substanz | Quellmittel | Polymer |
|---|---|---|
| Cyclophosphamid | Ethanol, Chloroform, Toluol, Aceton, Dioxan | PUR, PDMS, Poly-Polycarbonat, Polyethylen |
| Tamoxifen | Chloroform, Hexan | PDMS, PMMA, Polyethylen |
| Buselerin | Methanol, Ethanol | PUR, Polycarbonat, Cellulose |
| Ketoconazol | Chloroform, Methanol | PDMS, PMMA, Polycarbonat, Polyethylen |
| Diclofenac | DMF, Ethanol | PUR, Polycarbonat |
| Indometacin | Chloroform | PMDS, PMMA |
| Ibuprofen | Chloroform, Ethanol, Aceton, Ether | PMDS, PMMA, Teflon |
| Piroxicam | Methanol | PUR, Polycarbonat, Cellulose |
| Phenylbutazon | Chloroform, Aceton | PDMS, PMMA |
| Acetylsalicylsäure | Ethanol | PMMA, Polyester |
| Glukokortikoide (Steroide) | Ethanol, Methanol, Acton, Chloroform mit Ausnahme von Triaminolonacetonid, welches gut in DMF löslich ist | Zur Quellung können fast alle verwendbaren Polymere im Bereich mittlerer Kohäsionsenergiedichten verwendet werden, da es sich bei den Steroiden um amphiphile Stoffe handelt |
| Acetylsalicylsäure | Ethanol | PUR, PMMA, Polyester |
| Isosorbidnitrat | Chloroform, Aceton | PDMS, Teflon, PMMA |
| Nifedipin | Chloroform, Aceton | PDMS, Teflon, PMMA |
| Verapamil | Chloroform, Aceton, Ethylacetat | PDMS, Teflon, PMMA, Polyethylen |
| Nadolol | Methanol | PUR, Polycarbonat, Cellulose, Polyethylenterephthalat |
| Metropolol | H₂O, Ethanol | Cellulose, PUR |
| Pindolol | höhere Alkohole | PUR, Polycarbonat, Cellulose |

Weitere Informationen zur Auswahl geeigneter Partner sind in der EP-A-0 560 875 enthalten.

Auch Polyethylentherephthalat kann mit ausgesuchten Solventien, Aceton, Benzaldehyd, Dichlorethan, Diphenylether, Acetophenyl, THF, Dioxan, CHCl₃, CH₂Cl₂ in den Quellungszustand überführt werden. PET wird beispielsweise für künstliche Gefäße und Herzklappen eingesetzt.

Eine andere effiziente Methode beruht darauf, daß mit Hilfe von Lösungsmitteln Polymere aufgelöst, mit den pharmazeutischen Produkten vermischt, in eine Form gegeben werden und das Lösemittel evaporiert wird; dies nennt man "Solvent Casting". Die Technik des Solvent Casting ist lange bekannt. Erstmals 1991 konnte die Freisetzungskinetik gegen antimikrobielle Effizienz gezeigt werden (3). Dies war bei nicht-vernetzten Polymeren mit Lösemitteln möglich, die nichttoxisch sind und leicht evaporiert werden können. Es können damit sehr hohe Wirkstoff-Konzentrationen in dem Kunststoff erreicht werden (1, 3, 5, 7 bis 9). Zur Erzielung einer langen Abgabedauer ist insbesondere eine molekular-disperse Verteilung einer im Polymer löslichen Substanz geeignet (7). Oberflächlich gebundene Wirkstoffe sind weniger effizient (3, 8).

Eine weitere Möglichkeit zur Herstellung der erfindungsgemäßen Medizinprodukte besteht darin, pharmazeutisch aktive Gefäßprothesen in Form von Schläuchen oder dünnen Röhrchen durch Erspinnen von Fasern aus Polymerlösungen (spezielle Form des Solvent Casting) mit den oben genannten Wirkstoffen und Transportieren und Ablegen der Fasern auf einer stabförmigen Unterlage zu einem Vlies herzustellen. Hierbei können Polyamide, Polyacrylate und Polyurethane mit lipophilen oder hydrophilen Substanzen vereinigt werden.

Weitere Möglichkeiten zum Einbringen oder Befüllen von Kunststoffen mit pharmazeutischen Wirkstoffen bestehen in der Extrusion oder im thermischen Spritzguß von Polymeren. Hierbei ist es nur möglich, Wirkstoffe mit Polymeren zu kombinieren, deren Schmelzpunkte und thermische Belastbarkeit ähnlich hoch sind. Die Kombination von Wirkstoffen mit Kunststoffen wie Polyurethan, Silikon, Polyethylenterephthalat, Polyethylen und anderen hochschmelzenden Polymeren ist nur mit Wirkstoffen möglich, die einen ähnlich hohen Schmelzpunkt (bzw. thermische Stabilität) haben. Dies wird beispielsweise für Gentamicinsulfat offenbart. Niedriger schmelzende Kunststoffe für die Schmelzextrusion und den Spritzguß können dabei verwendet werden. Vorzugsweise sind es Polymere, welche in einem Bereich von 100°C ± 30% thermoplastisch verarbeitbar sind. Weiterhin ist es möglich, den Schmelzpunkt von Polymeren mittels CO₂ Begasung bei Extrusion zu senken und somit thermolabile Stoffe hinzuzugeben.

Soweit die Substanz B antimikrobielle Eigenschaften haben soll, hat es sich gezeigt, daß Rifampicin intrazellulär Bakterien penetriert und eine effiziente Substanz gegen Biofilme darstellt (2). Auch andere Ansamycinderivate mit ähnlichen Eigenschaften sind denkbar (Rifamycin, Rifapentin). Vorzugsweise sind antimikrobielle Substanzen mit lipophilen Eigenschaften genannt, welche auch klinischen Nutzen gefunden haben für sogenannte "Difficult to Treat Infections". Denkbar sind grundsätzlich alle antimikrobiell wirksamen Gruppen wie lipophile Vertreter aus der Gruppe der Aminoglykoside, aus der Gruppe der Cephalosporine und darauf bezogenen Betalactame, des Chloramphenicols, Lincosamiden, Makroliden, Penicillinen, Chinolonen, Sulfonamiden, Tetracyclinen. Vorzugsweise lipophile Antibiotika sind Benzathin, Phenoxymethylpenicillin, Chloramphenicol, Chlortetracydin, Ciprofloxacin-Betain, Clarithromycin, Clindamycin-Palmitathydrochlorid. Trimethoprim, Erythromycin-2-Acetat, -Stearat; Erythromycin-Estolat, Erythromycin-Ethylsuccinat-, Erythromycin-Glutamat, -Laktopropionat, -Stearat, Fusidinsäure, vorzugsweise freie Fusidinsäure, Gramicidin, Mupirocin, lipophile Vertreter der Imidazolreihe wie Econazol, Itraconazol, Clotrimazol und andere, Pristinamycin, Silbersulfadiazin, ausgenommen in der Kombination Silbersulfadiazin-Chlorhexidin, Sparfloxacin, Teicoplanin bzw. Ramoplanin, Pristinamycin wegen des lipophilen Alkylrestes und Tyrothricin wegen der Wasserunlöslichkeit.

Besonders erwähnenswert sind Miconazol-Salizylat, Sulfosalicyl-Miconazol bzw. Miconazol-Acetylsalicylat als lipophile Komponente; welche aus der Miconazol-Base präpariert werden können. Diese Substanzen sind vorteilhaft, weil sie antimikrobiell und antithrombogen wirksam sind.
- **Figur 1:**: Freisetzung Rifampicin-Clotrimazol-Katheter

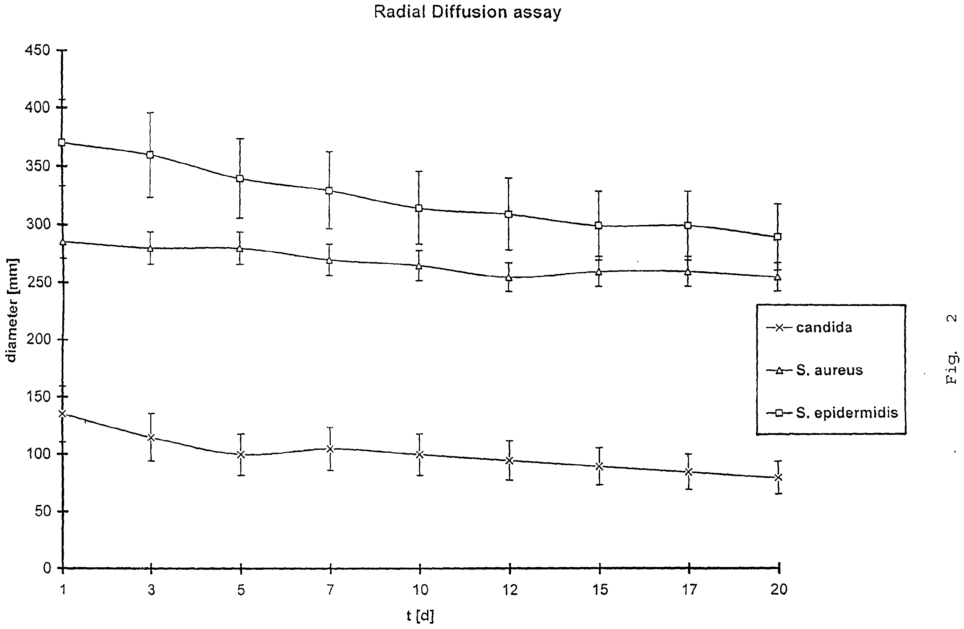

- **Figur 2:**: Antimikrobielle Halbwertzeit im Agar-Diffusionstest gegen unterschiedliche Bakterienspezies

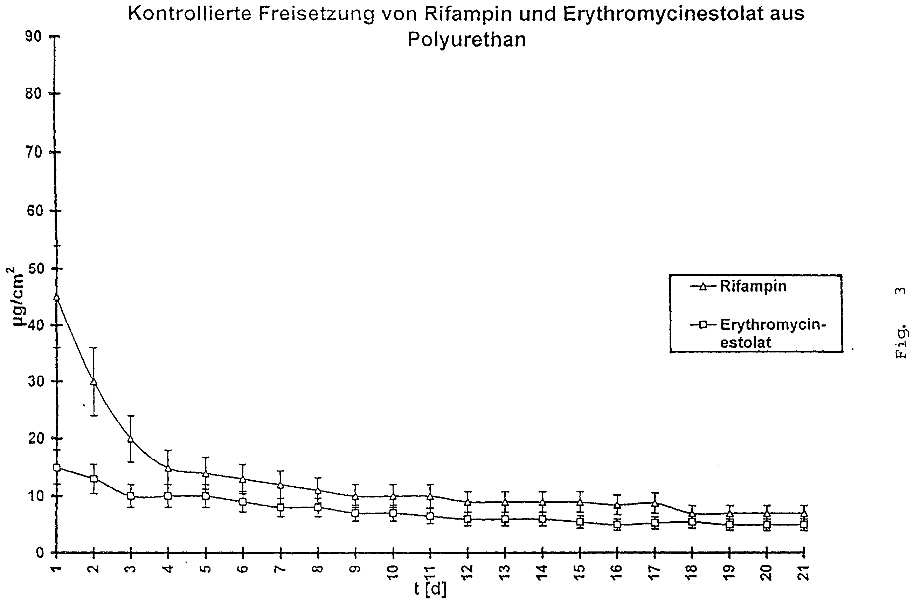

- **Figur 3:**: Freisetzung Rifampicin-Erythromycin-Estolat

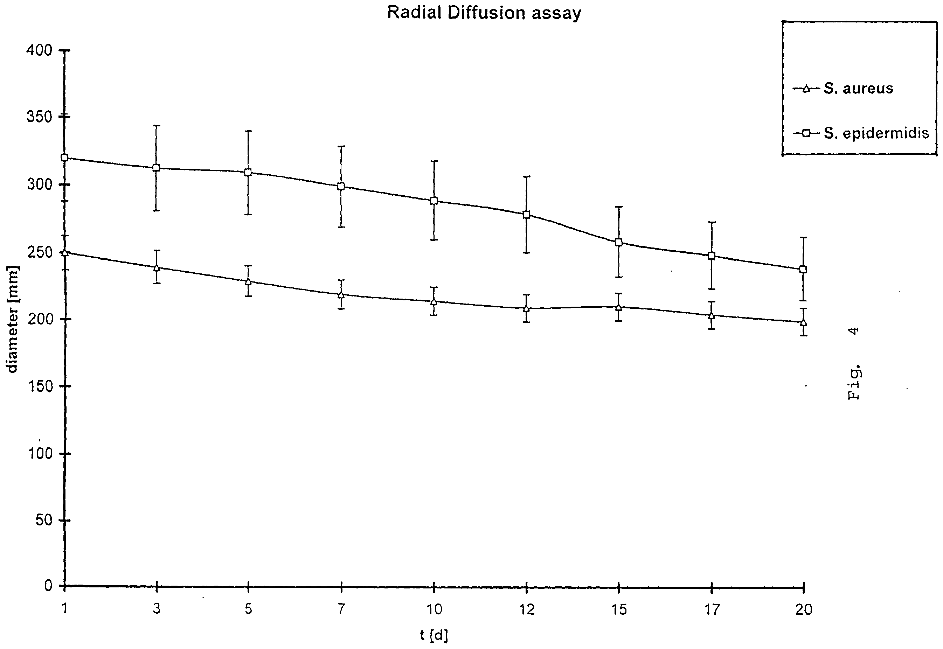

- **Figur 4:**: Antimikrobielle Halbwertzeit im Agardiffusionstest (Staphylokokken)

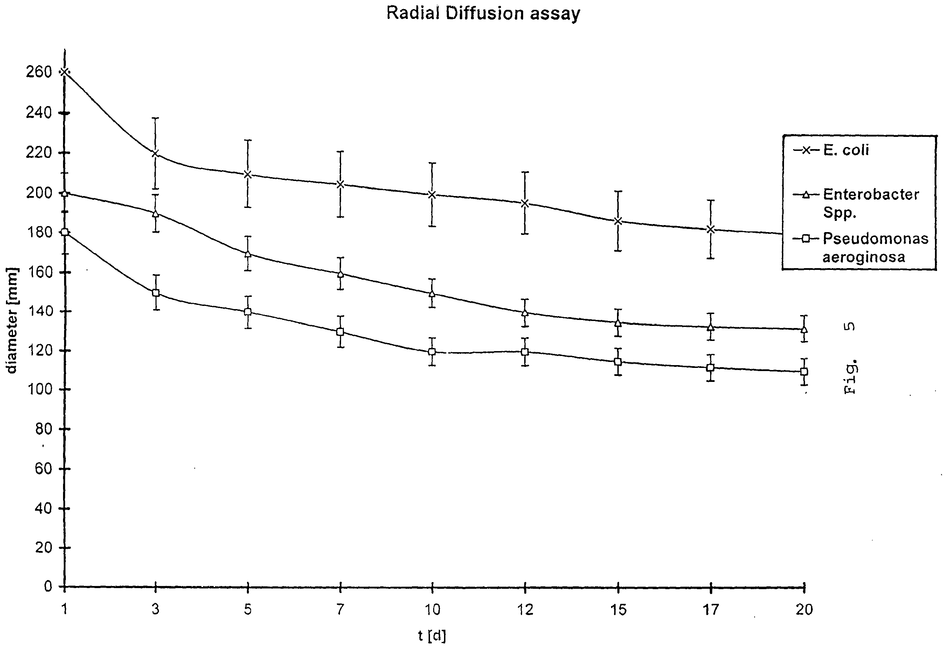

- **Figur 5:**: Antimikrobielle Halbwertzeit im Agardiffusionstest (Enterobactericeae)

Die Erfindung wird anhand der folgenden Beispiele näher erläutert.

### Beispiel 1 (Referenz)

Es wird die Herstellung eines Polyurethanfilms beschrieben, welcher Antitumoragentien enthält, welche mittels kontrollierter Freisetzung aus dem medizinischen Kunststoff freigesetzt werden, zu welchem lipophile Substanzen zugesetzt werden, um die Freisetzung zu retardieren. Hier werden Matrix-, Metalloproteinasen bzw. Combretastatin bzw. Vascular-epithelial growth factor bzw. Thalidomid bzw. Squalamin oder SU5416 ein Tyrosinkinaseenzymblocker in einem Verhältnis von 5 Gew.-% zu 5 Gew.-% Miconazolnitrat per Solvent casting in Tetrahydrofuran und Pellethane (10% Pellethane, 1 % Substanzen, 89% Solvens) gelöst. Das Polymere wird mit der Substanzmischung in eine Form gegossen, bei 40°C langsam evaporiert, so daß das Lösemittel vollständig abgedampft wird und ein Film entsteht. Die Freisetzung dieser Substanzen, gemessen per HPLC, überschreitet 30 Tage. Nach zwei Tagen erhält man ein steady state release von 2 bis 5 µg pro Substanz pro cm² Polymeroberfläche.

### Beispiel 2

Ein Polyurethankatheter wird in Essigsäureethylester mit 5% (w/w) Rifampicin und 10% (w/w) Clotrimazol bei 30°C 10 Minuten gequollen, so daß die Substanzen in die aufgedehnte polymere Matrix hineindiffundieren können. Danach wird der Katheter aus der Lösung herausgenommen, bei Raumtemperatur 24 Stunden evaporiert, danach bei Unterdruck 48 Stunden und 50°C evaporiert, um sämtliches Restlösemittel aus dem Kunststoff herauszuziehen.

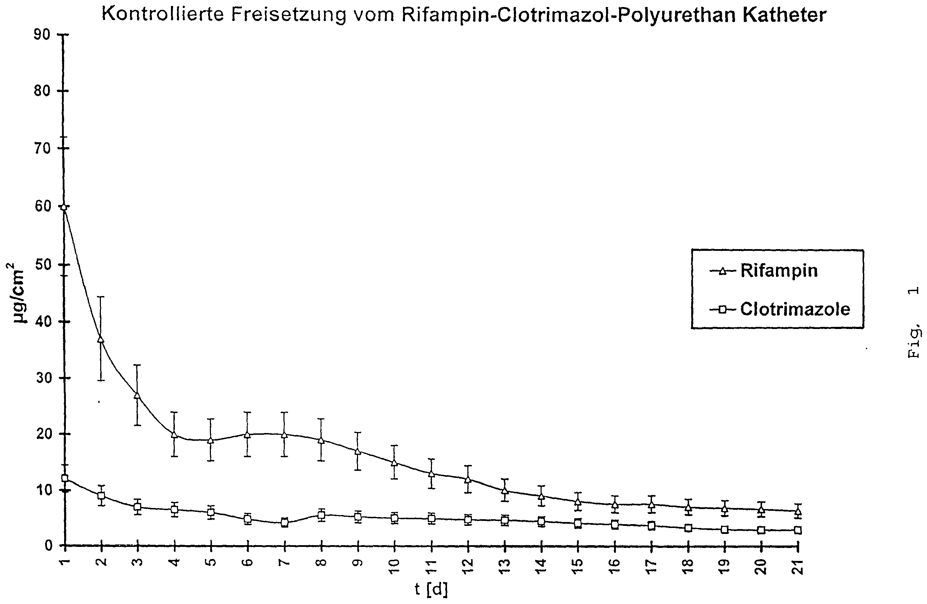

Diese Rifampicin-Clotrimazol-Polyurethanbeschichtung ist in der Lage, mehr als zwei Monate lang effizient Wirkstoff abzugeben. Die Freisetzungskurve von Clotrimazol-Rifampicin ist in Figur 1 dargestellt, in welcher das sehr viel besser wasserlösliche Rifampicin nach dem initialen Burst-Effekt oberflächlich gebundene Wirkstoffe durch den lipophilen Retentionseffekt von Clotrimazol ein ähnliches konstantes Freisetzungsmuster nahezu nullter Ordnung wie Clotrimazol erhält. Dies ist nur dadurch möglich, daß dies sehr schwer wasserlösliche Clotrimazol als Reservoir, Kotransporter und gleichzeitig aber als Retentionsmembran für das hydrophilere Rifampicin fungiert. Dies ist auch mit anderen Substanzen, beispielsweise Fenticonazol oder anderen Imidazol-Derivaten wie Miconazol, Sulfosalicyl-Miconazol, Miconazolnitrat, Miconazolsalicylat bzw. Miconazolacetylsalicylat und anderen Miconazol-Derivaten möglich. Auch andere Imidazol-Derivate, welche fast wasserunlöslich sind, wie Ketoconazol, Itraconazol, Fenticonazol, Econazol und andere sind denkbar. Nach drei Wochen Freisetzungsdauer sind, noch > 80% des Wirkstoffs im Implantatmaterial vorhanden. Das Freisetzungsprofil kann auf mehr als drei Monaten approximiert werden.

### Beispiel 3 (Referenz)

### Präparationen eines Polyurethan-Katheters mittels Dichlormethan, Rifampicin und einem lipophilen Erythromycinsalz (Erythromycin-Estolat)

Das lipophile Erythromycinsalz ist in wäßrigen Lösungen fast unlöslich im Gegensatz zu dem mäßig wasserlöslichen Rifampicin. Es erfolgte eine Quellung einer 5%igen Rifampicin-, 10%igen Erythromycinlösung mit Pelethane-Kathetern in Dichlormethan 15 min. bei 30°C, Trocknung des gequollenen Katheters 24 Stunden bei Raumtemperatur, dann Evaporieren bei Unterdruck 0,1 mbar, 50°C für 48 Stunden. Hierbei entsteht ein Slow Release-System mit einem Inkorporierungsgehalt von 10 Massenprozent, in welchem nach dem Burst-Effekt die Freisetzung von Rifampicin ähnlich wie in Beispiel 2 parallel zu der langsamen Freisetzung des lipophilen Gentamicinsalzes verläuft. Hierbei wirkt dieses lipophile Erythromycinsalz wiederum als interne Diffusionsmembran.

### Beispiel 4 (Referenz)

### Inkorporierung der amphiphilen Chlorhexidin-base und des lipophilen Clindamycin-Palmitats in Polyurethan mittels Solvent Casting

Hierbei wird das Polyurethan in Tetrahydrofuran aufgelöst und in diese Lösung jeweils 5% Chlorhexidin-base und 5% des schwerlöslichen Clindamycinsalzes hinzugegeben. Nach Rühren und Temperaturerhöhung auf 60°C wird diese Lösung in eine Form gegeben, das Lösemittel langsam bei Unterdruck abgedampft und später bei 50° C und Unterdruck vollständig evaporiert. Dieser Wirkstoff-enthaltene Polyurethanfilm war in der Lage, mehr als zwei Monate Wirkstoffe freizusetzen, wobei das lipophile Clindamycinsalz die Abgabe von Chlorhexidinbase retardierte und zu einer Freisetzung nahezu nullter Ordnung führte.

### Beispiel 5 (Referenz)

### Beschichtung einer Totalendoprothese mit einer Miconazalbase-Rifampicin Mischung

Eine Totalendoprothese, beschichtet mit einer 200 µ dicken, porösen Hydroxy-Apatit-Schicht wurde in eine 5% Rifampicin, 10% Miconazol-Base enthaltende Aceton-Lösung für 5 Minuten gegeben. Daraufhin wurde die Prothese herausgenommen und bei Raumtemperatur 24 Stunden getrocknet. Um die Trägerschicht zu vergrößern, wurde die Prothese noch zweimal 5 Sekunden in das Bad getaucht und getrocknet. Die beschichtete Prothese wurde innerhalb von 20 Tagen in Phosphatpuffer bei 37°C eluiert. Miconazol-Base wirkte hier als Reservoir und Träger für das hydrophilere Rifampicin. Auch die Kombination Rifampicin mit dem schwerlöslichen Ciprofloxacin-betain war 14 Tage freisetzungsaktiv (nicht gezeigt).

### Beispiel 6 (Referenz)

### Polyurethan-Harnwegskatheter mit EDTA-Säure / Clindamycin-Beschichtung zur Verhinderung der Inkrustation und Infektion

Eine 10%ige Lösung von Polyurethan in Tetrahydrofuran wurde mit einer 10%igen (bezogen auf das Polymer) Lösung von EDTA und Clindamycin-Palmitat versetzt. Die Lösung wurde wie in Beispiel 4 in eine Form gegeben und evaporiert. Über einen Zeitraum von vier Wochen konnte per HPLC eine Abgabe größer 2 µg/cm² Oberfläche gemessen werden.

### Beispiel 7 (Referenz)

### Polyurethan-Harnwegskatheter mit Ag-Salizylat und Tioconazol

Eine Lösung aus 90% Tetrahydrofuran, 9% PUR, 0,5% Ag-Salizylat und 0,5% Tioconazol wurde bei 40°C zur Homogenität verührt. Ein Polyurethankatheter wurde sukzessiv für jeweils 20 Sekunden in die Lösung gedippt und getrocknet. Diese Prozedur wurde 5 mal wiederholt. Nach dem zweiten Tag der Elution zeigte der beschichtete Katheter über einen Zeitraum von 30 Tagen eine Freisetzung nahezu nullter Ordnung.

### Literatur

(1) Olanoff LS, Anderson JM, Jones RD: Sustained release of gentamicin from prosthetic heart valves. Trans. Am. Soc. Artif. Intern. Organs 1979; 25: 334 - 338.
(2) Schierholz JM, Jansen B, Jaenicke L, Pulverer G: In vitro efficacy of an antibiotic releasing silicone ventricle catheter to prevent shunt infection. Biomat. 1994; 15: 996 - 1000.
(3) Schierholz JM, Jansen B, Steinhauser H, Peters G, Schuhmacher-Perdreau F, Pulverer G: Drug release from antibiotic-containing polyurethanes. New Polymeric Mater 1990; 3: 61 - 72.
(4) Schierholz JM, Rump AFE, Pulverer G: Drug delivery concepts for efficacious prevention of foreign-body infections. Zbl. Bakt. 1996; 284: 390-401.
(5) Schierholz JM, Rump AFE, Pulverer G: Ciprofloxacin containing polyurethanes as potential drug delivery systems to prevent foreign-body infections. Drug Res. 1997; 47 (I), 1: 70-74
(6) Schierholz JM: Physico-chemical properties of a rifampicin-releasing polydimethyl-siloxane shunt. Biomat. 17: 1997
(7) Schierholz JM, Steinhauser H, Rump AFE, Berkels, R, Pulverer G: Controlled release of antibiotics from biomedical polyurethanes: morphological and structural features. Biomat. 18: 1997
(8) Sherertz RJ, Carruth WA, Hampton AA, Byron MP, Solomon DD: Efficacy of antibiotic-coated catheters in preventing subcutaneous staphylococcus aureus infection in rabbits. J. Inf. Dis. 1993; 167: 98 - 106.
(9) Solomon DD, Sherertz RJ: Antibiotic releasing polymers. J. Contr. Rel. 1987: 343 - 352.

## Patentansprüche

1. Nicht abbaubares Medizinprodukt mit einer nicht abbaubaren polymeren Matrix als einem Trägermaterial und einer monolithischen Verteilung von zwei Substanzen mit unterschiedlicher Hydrophilie, von denen eine erste als Substanz A und eine zweite als Substanz B bezeichnet wird, die Substanz A lipophiler ist als die Substanz B, Substanz A eine Löslichkeit (w/w) in Wasser bei 25°C von 300. µg/ml bis 1 µg/ml aufweist, Substanz B eine höhere Löslichkeit aufweist als Substanz A, zumindest eine der Substanzen A und B ein pharmazeutischer Wirkstoff ist und wobei die Menge an Substanz A und B jeweils zwischen mindestens einer wirksamen Menge bis hin zu 10 Gew. % , bezogen auf das Gewicht des Trägermaterials vorhanden ist
ausgenommen
die Kombinationen Chlorhexidin/Silbersulfadiazin, Triclosan-Chlorhexidin, Polyethylenglycol-Polyurethan oder mit Kombinationen von Clotrimazol und Triclosan oder poröses Polyethylen mit Kombinationen von Clotrimazol und Triclosan und
**dadurch gekennzeichnet, daß** die Substanz A ausgewählt wird aus den lipophilen Vertretern der Imidazolreihe wie Econazol, Itraconazol, Clotrimazol, Pristinamycin, Sulfosalicyl-Miconazol, Miconazol-Salizylat bzw. MiconazolAcetylsalicylat, Miconazol, Miconazolnitrat, Miconazolderivaten, Miconazol-Base Ticlopidin, Argatroban (CAS 73963-72-1), Cloricromen (CAS 68206-94-0), Clorindion (CAS 1146-99-2), Nafazatram, Triflusal und Dipyridamol.

2. Medizinprodukt gemäß Anspruch 1, **dadurch gekennzeichnet, daß** die Substanz B ein pharmazeutischer Wirkstoff ist.

3. Medizinprodukt gemäß mindestens einem der Ansprüche 1 bis 2, **dadurch gekennzeichnet, daß** das Medizinprodukt aus einem Material besteht, das ausgewählt wird aus Siloxan, Polyurethan, Acrylaten, Polycarbonaten, Cellulose, Cellulose-Derivaten, Polymeren aus Tetrafluorethylen und Polyethylentherephthalat und Hydrogele.

4. Medizinprodukt gemäß mindestens einem der Ansprüche 1 bis 3, **dadurch gekennzeichnet, daß** die Substanz B ausgewählt wird aus Phospholipiden und Hyaluronsäurederivaten, nicht-ionische Surfactants wie Acetoylglyceride, Diacetyglyceride, Monoglyceride Diethylen-Glycol-Monostearat, Ethylen-Glycol-Monostearat, Glycol-Monostearat, Verbindungen von Monolaurylethern, Mono-DiGlyceride, Propylen-Glycol-Monostearat; Sorbit-Monostearat, -Palmitat, - Sesquioleat, -tridecanat, -Tristearat und deren hydrophilen Derivaten, des Chloramphenicols, Lincosamiden, Makroliden, Penicillinen, Chinolonen, Sulfonamiden, lipophile Antibiotika wie Benzathin, Phenoxymethylpenicillin, Chloramphenicol, Chlortetracyclin, Clarithromycin, Clindamycin-Palmitathydrochlorid Trimethoprim, Erythromycin-2-Acetat, -Stearat; Erythromycin-Estolat, Erythromycin-Ethylsuccinat-, Erythromycin-Glutamat, - Laktopropionat und die Erythromycin, -Stearat, Fusidinsäure, Gramicidin, Mupirocin, lipophile und hydrophile Vertreter der Imidazolreihe wie Econazol, Itraconazol, Clotrimazol und deren hydrophile Vertreter, Pristinamycin, Rifabutin, Rifapentin, Rifampicin und anderen hydrophilen Staphylokokken wirksame Antibiotika, Ticlopidin, Argatroban (CAS 73963-72-1), Cloricromen (CAS 68206-94-0), Clorindion (CAS 1146-99-2), Nafazatram, Triflusal und Dipyridamol.

5. Medizinprodukt gemäß mindestens einem der Ansprüche 1 bis 4, **dadurch gekennzeichnet, daß** der Wirkstoff durch kovalente oder nicht-kovalente Modifikation, wie Veresterung, Etherbildung, Acetal-, Halbacetalbildung, Ringbildung, Adduktbildung, Komplexbildung und/oder Salzbildung mit einem lipophilen Gegenion lipophilisiert wurde.

6. Medizinprodukt nach Anspruch 5, **dadurch gekennzeichnet, dass** die Komplexbildung und/oder Salzbildung durch die Assoziation zweier Wirkstoffe erfolgt.

7. Medizinprodukt nach einem der Ansprüche 1 bis 6, **dadurch gekennzeichnet, dass** die lipophile Substanz zusätzlich oberflächlich angelagert ist.

8. Medizinprodukt gemäß mindestens einem der Ansprüche 1 bis 7, **dadurch gekennzeichnet, daß** sich der pharmazeutische Wirkstoff intrazellulär in Bakterien, Thrombozyten und/oder anderen Zelltypen anreichern kann.

9. Medizinprodukt gemäß mindestens einem der Ansprüche 1 bis 8, **dadurch gekennzeichnet, daß** das Medizinprodukt eine Augenlinse, ein Katheter, eine Gefäßprothese, eine Endoprothese, ein chirurgisch antimikrobieller Wirkstoffträger wie ein Kollagenvlies, Stents, Blades, Knochenzement, metallische Endoprothesen, CAPD-Katheter, Wundauflagen, gesprühte Polyurethanvliese, Drainage ist.

10. Verfahren zur Herstellung von Medizinprodukten gemäß mindestens einem der Ansprüche 1 bis 9, **dadurch gekennzeichnet, daß** sie über Quellung, Solvent Casting, Wirkstofflackierung, Extrusion und/oder Spritzguß von Polymeren wie PUR, SIR, PET erhalten werden.

11. Verfahren zur Retardierung der Freisetzung einer Substanz B aus einem Medizinprodukt gemäß einem der Ansprüche 1 bis 9, **dadurch gekennzeichnet, daß** Substanz B zusammen mit einer Substanz A in das Medizinprodukt gebracht wird, wobei Substanz A eine Löslichkeit (w/w) in Wasser von 300 µg/ml bis 1 µg/ml aufweist, Substanz B eine höhere Löslichkeit aufweist als Substanz A und zumindest eine der Substanzen A und B ein pharmazeutischer Wirkstoff ist.

## Claims

1. A non-degradable medical product with a non-degradable polymeric matrix as a support material and a monolithic distribution of two substances having different degrees of hydrophilicity, of which the first is referred to as substance A and the second is referred to as substance B, substance A being more lipophilic than substance B, wherein substance A has a solubility (w/w) in water at 25 °C of from 300 µg/ml to 1 µg/ml, substance B has a higher solubility than that of substance A, at least one of substances A and B is a pharmaceutically active substance, and wherein the amount of substance A and B is respectively from at least an effective amount to 10% by weight, based on the weight of the support material;
with the exception of the combinations chlorohexidine/silver sulfadiazine, triclosane/chlorohexidine, polyethylene glycol/polyurethane, or with combinations of clotrimazole and triclosane, or porous polyethylene with combinations of clotrimazole and triclosane,
**characterized in that** substance A is selected from the lipophilic members of the imidazole series, such as econazole, itraconazole, clotrimazole, pristinamycin, sulfosalicylmiconazole, miconazole salicylate and miconazole acetylsalicylate, miconazole, miconazole nitrate, miconazole derivatives, miconazole base, ticlopidin, argatroban (CAS 73963-72-1), cloricromene (CAS 68206-94-0), clorindione (CAS 1146-99-2), nafazatram, triflusal and dipyridamole.

2. The medical product according to claim 1, **characterized in that** substance B is a pharmaceutically active substance.

3. The medical product according to at least one of claims 1 to 2, **characterized in that** said medical product is made of a material selected from siloxane, polyurethane, acrylates, polycarbonates, cellulose, cellulose derivatives, polymers of tetrafluoroethylene, polyethylene terephthalate and hydrogels.

4. The medical product according to at least one of claims 1 to 3, **characterized in that** substance B is selected from phospholipids and hyaluronic acid derivatives, non-ionic surfactants, such as acetylglycerides, diacetylglyceride, monoglycerides, diethylene glycol monostearate, ethylene glycol monostearate, glycol monostearate, compounds of monolauryl ethers, mono-di-glycerides, propylene glycol monostearate, sorbitol monostearate, sorbitol palmitate, sorbitol sesquioleate, sorbitol tridecanate, sorbitol tristearate and their hydrophilic derivatives, chloramphenicol, lincosamides, macrolides, penicillins, quinolones, sulfonamides, lipophilic antibiotics, such as benzathin, phenoxymethylpenicillin, chloramphenicol, chlortetracyclin, clarithromycin, clindamycin palmitate hydrochloride, trimethoprim, erythromycin 2-acetate, erythromycin 2-stearate; erythromycin estolate, erythromycin ethylsuccinate, erythromycin glutamate, erythromycin lactopropionate, and erythromycin, erythromycin stearate, fusidinic acid, gramicidin, mupirocin, lipophilic and hydrophilic members of the imidazole series, such as econazole, itraconazole, clotrimazole and their hydrophilic members, pristinamycin, rifabutin, rifapentin, rifampicin and other hydrophilic staphylococci-effective antibiotics, ticlopidin, argatroban (CAS 73963-72-1), cloricromene (CAS 68206-94-0), clorindione (CAS 1146-99-2), nafazatram, triflusal and dipyridamole.

5. The medical product according to at least one of claims 1 to 4, **characterized in that** the active substance is lipophilized by covalent or non-covalent modification, such as esterification, ether formation, acetal or semiacetal formation, ring formation, adduct formation, complex formation and/or salt formation with a lipophilic counter-ion.

6. The medical product according to claim 5, **characterized in that** said complex formation and/or salt formation is effected through the association of two active substances.

7. The medical product according to any of claims 1 to 6, **characterized in that** said lipophilic substance is additionally deposited on the surface.

8. The medical product according to at least one of claims 1 to 7, **characterized in that** the pharmaceutically active substance is capable of becoming intracellularly enriched in bacteria, platelets, and/or other cell types.

9. The medical product according to at least one of claims 1 to 8, **characterized in that** the medical product is an eye lens, catheter, vascular prosthesis, endoprosthesis, surgical antimicrobial drug carrier, such as collagen web, stents, blades, bone cement, metallic endoprostheses, CAPD catheters, wound plasters, sprayed polyurethane webs, drains.

10. A process for the preparation of medical products according to at least one of claims 1 to 9, **characterized in that** they are obtained by swelling, solvent casting, active substance coating, extrusion and/or injection molding of polymers, such as PUR, SIR, PET.

11. A method for retardation of the release of a substance B from a medical product according to any of claims 1 to 9, **characterized in that** substance B is incorporated in said medical product together with a substance A, wherein substance A has a solubility (w/w) in water of from 300 µg/ml to 1 µg/ml, substance B has a higher solubility than that of substance A, and at least one of substances A and B is a pharmaceutically active substance.

## Revendications

1. Produit à usage médical non délitable avec une matrice polymérique non délitable comme substrat et une répartition monolithique de deux substances ayant une hydrophilie différente, parmi lesquel(le)s une première substance est décrite comme étant la substance A et une seconde substance étant décrite comme étant la substance B, la substance A étant plus lipophile que la substance B, la substance A présentant une solubilité (p/p) dans l'eau à 25 °C de 300 µg/ml à 1 µg/ml, la substance B présentant une solubilité plus élevée que la substance A, au moins l'une des substances A et B étant un principe actif pharmaceutique et les quantités de substances A et B étant respectivement comprises entre au moins une quantité efficace jusqu'à 10 % en poids sur la base du poids du substrat, à l'exception des combinaisons chlorhexidine/sulfadiazine argentique, triclosan/chlorhexidine, polyéthylène glycolpolyuréthane ou avec des combinaisons de clotrimazole et de triclosan ou de polyéthylène poreux avec des combinaisons de clotrimazole et de triclosan,
**caractérisé en ce que** la substance A est choisie parmi les représentants lipophiles de la série des imidazoles comme l'éconazole, l'itraconazole, le clotrimazole, la pristinamycine, le miconazole sulfosalicylique, le salicylate de miconazole, ou l'acétylsalicylate de miconazole, le miconazole, le nitrate de miconazole, des dérivés de miconazole, la base de miconazole, la ticlopidine, l'argatroban (CAS 73963-72-1), le cloricromène (CAS 68206-94-0), la clorindione (CAS 1146-99-2), le nafazatram, le triflusal et le dipyridamol.

2. Produit à usage médical selon la revendication 1, **caractérisé en ce que** la substance B est un principe actif pharmaceutique.

3. Produit à usage médical selon au moins l'une des revendications 1 à 2, **caractérisé en ce que** le produit à usage médical est constitué d'une substance qui est choisie parmi le siloxane, le polyuréthane, les acrylates, les polycarbonates, la cellulose, les dérivés de la cellulose, les polymères de tétrafluoréthylènes et le polyéthylène téréphtalate et les hydrogels.

4. Produit à usage médical selon au moins l'une des revendications 1 à 3, **caractérisé en ce que** la substance B est choisie parmi les phospholipides et les dérivés de l'acide hyaluronique, des tensioactifs non ioniques comme les acétylglycérides, les diacétylglycérides, les monoglycérides, le monostéarate de diéthylène glycol, le monostéarate d'éthylène glycol, le monostéarate de glycol, les composés de monolauryléthers, les monoglycérides, les diglycérides, le monostéarate de propylène glycol ; le monostéarate de sorbitol, le palmitate de sorbitol, le sesquioléate de sorbitol, le tridécanate de sorbitol, le tristéarate de sorbitol et leurs dérivés hydrophiles, du chloramphénicol, des lincosamides, des macrolides, des pénicillines, des quinolones, des sulfonamides, des antibiotiques lipophiles comme la benzathine, la phénoxyméthylpénicilline, le chloramphénicol, la chlorotétracycline ; la clarithromycine, le chlorhydrate de palmitateclindamycine, le triméthoprim, du 2-acétate d'érythromycine, du stéarate d'érythromycine ; de l'estolate d'érythromycine, de l'éthylsuccinate d'érythromycine, du glutamate d'érythromycine, du lactopropionate d'érythromycine et l'érythromycine, le stéarate d'érythromycine, l'acide fusidique, la gramicidine, la mupirocine, les représentants lipophiles et hydrophiles de la série des imidazoles comme l'éconazole, l'itraconazole, le clotrimazole et leurs représentants hydrophiles, la pristinamycine, la rifabutine, la rifapentine, la rifampicine et d'autres antibiotiques hydrophiles antistaphylococciques, la ticlopidine, l'argatroban (CAS 73963-72-1), le cloricromène (CAS 68206-94-0), la clorindione (CAS 1146-99-2), le nafazatram, le triflusal et le dipyridamol.

5. Produit à usage médical selon au moins l'une des revendications 1 à 4, **caractérisé en ce que** le principe actif a été lipophilisé par modification covalente ou non covalente comme une estérification, une éthérification, une acétalisation, une hémiacétalisation, une cyclisation, une formation d'adduits, une complexion et/ou une salification avec un contre-ion lipophile.

6. Produit à usage médical selon la revendication 5, **caractérisé en ce que** la complexion et/ou la salification se produit par l'association de deux principes actifs.

7. Produit à usage médical selon l'une des revendications 1 à 6, **caractérisé en ce que** la substance lipophile est ajoutée de plus de manière superficielle.

8. Produit à usage médical selon au moins l'une des revendications 1 à 7, **caractérisé en ce que** le principe actif pharmaceutique peut se concentrer de manière intracellulaire dans des bactéries, des thrombocytes et/ou d'autres types de cellules.

9. Produit à usage médical selon au moins l'une des revendications 1 à 8, **caractérisé en ce que** le produit à usage médical est un cristallin, un cathéter, une prothèse vasculaire, une endoprothèse, un support de principe actif antimicrobien chirurgical comme un voile de collagène, des stents, des lames, un ciment osseux, des endoprothèses métalliques, des cathéters pour DPAC, des pansements, des voiles de polyuréthane pulvérisés, un drainage.

10. Procédé pour fabriquer des produits à usage médical selon au moins l'une des revendications 1 à 9, **caractérisé en ce qu'**ils peuvent être obtenus avec un revêtement de principe actif par gonflement, coulée de solvant, enrobage de principe actif, extrusion et/ou injection de polymères comme PUR, SIR, PET.

11. Procédé pour retarder la libération d'une substance B à partir d'un produit à usage médical selon l'une des revendications 1 à 9, **caractérisé en ce que** la substance B est introduite dans le produit a usage médical conjointement avec une substance A, la substance A présentant une solubilité (p/p) dans l'eau de 300 µg/ml à 1 µ/ml, la substance B présentant une solubilité supérieure à celle de la substance A et au moins l'une des substances A et B étant un principe actif pharmaceutique.
